# EUROPÄISCHE PATENTSCHRIFT

(11) **EP 0 997 105 B1**
(45) Veröffentlichungstag und Bekanntmachung des Hinweises auf die Patenterteilung: **01.06.2005**
(21) Anmeldenummer: 98120232.8
(22) Anmeldetag: 26.10.1998
(51) Int. Cl.: A61B 17/32, A61F 9/007, A61M 1/00, A61M 39/02, A61B 17/22

(54) **Vorrichtung zum Entfernen von Krankheitsherden in der Human- und Veterinärmedizin**
Apparatus for removal of focus of disease in human and veterinary medicine
Dispositif pour écarter un foyer de maladie dans la médecine humaine et vétérinaire

(43) Veröffentlichungstag der Anmeldung: 03.05.2000
(73) Patentinhaber: Human Med AG, 19061 Schwerin (DE)
(72) Erfinder: Pein, Andreas, 19061 Schwerin (DE)
(74) Vertreter: Jaap, Reinhard

(56) Entgegenhaltungen:
- EP-A- 0 411 170
- EP-A- 0 657 150
- WO-A-98/07398
- DE-A- 4 018 736
- US-A- 3 882 872
- US-A- 4 014 333
- US-A- 4 573 979
- US-A- 5 788 667

## Beschreibung

Die Erfindung bezieht sich auf eine Vorrichtung zum Entfernen eines Krankeitsherdes nach den Oberbegriffen der Ansprüche 1.
Derartige Vorrichtungen werden in der Human- und Veterinärmedizin eingesetzt, wobei als Krankheitsherde körperliche Beeinträchtigungen und Mängel im weitesten Sinne anzusehen sind.

Krankeitsherde der angesprochenden Art befinden sich im Gehirn und im zentralen Nervensystem einschließlich der Augen sowie in jeglichen Körperstrukturen, deren Behandlung auf Grund ihrer Ennervierung, Vaskularisierung oder anderer Gegebenheiten eine besonders schonende Form des operativen Eingriffs wünschenswert macht. Nach bisherigen fachmedizinischen Kenntnissen werden solche Krankheitsherde mit chirugischen und mikrochirurgischen Mitteln behandelt oder abgetragen, die sich durch eine ganz oder überwiegend nach vorne gerichtete Krafteinwirkung kennzeichnen, z.B. Skalpelle jeglicher Art, Koagulatoren und Laser jeglicher Art, Ultraschall-Aspiratoren etc.. So ein Krankheitsherd oder degenerative Beeinträchtigung ist beispielsweise die vom Grauen Star beeinträchtigte Linse des Auges, die entfernt und gegen eine künstliche Linse ausgetauscht werden muß. Dazu wird zunächst das Auge mit einem Tunnelschnitt durchtrennt und anschließend die Vorderkapsel durch einen möglichst kreisrunden intakten Kapsulorhexis geöffnet. Durch diese Öffnung wird ein Instrument in die erkrankte Linse geschoben. Vornehmlich durch Ultraschall wird dann die Linse zunächst in kleine Bestandteile zertrümmert und anschließend abgesaugt. Nachdem alle Bestandteile der Linse entfernt sind und die Augenkammer gesäubert ist, wird eine künstliche Linse durch den Kanal eines Spezialinstrumentes in die Augenkammer eingeschoben. Nach dem Verlassen des Instrumentes entspannt sich die Linse und nimmt wieder ihre ursprüngliche Form einer Linse an. Zum Schluß wird die künstliche Linse ausgerichtet und verankert.
Obwohl diese Operationsmethode inzwischen zu einem Routineeingriff geworden ist, treten immer noch Komplikationen auf. So gelingt es nicht immer, die Augenkammer vollständig von Restbestandteilen der erkrankten Linse zu befreien, da einige der peripheren Anteile der erkrankten Zellreste dem Einblick des Chirurgen verborgen bleiben und daher vor Ort verbleiben. Dadurch besteht die Gefahr des Nachwachsens des grauen Stars. Die Zellreste durch eine manuelle Injektion von Flüssigkeiten zu mobilisieren, ist nur partiell möglich.
Zum Anderen entsteht durch die Ultraschallenergie überschüssige Wärme, die zur Erhitzung des Hornhautgewebes und damit zum Verlust von Endothelzellen führt.
Diese Operationsmethode erfordert auch eine Vielzahl von unterschiedlichsten Operationswerkzeugen. Dazu müssen auch viele einzelne und damit zeitraubende Handgriffe durchgeführt werden. Das alles verteuert diese Operationsmethode. Die Vielzahl der Operationsschritte und die Vielzahl an Operationswerkzeugen stellen auch hohe Anforderungen an den Chirurgen. Dadurch ist der Erfolg einer solchen Operationsmethode im starken Maße von der Qualifikation des Chirurgen abhängig.
Aus der WO 98/07398 Al und in ähnlicher Weise auch aus der US 4,573,979 ist nun eine Vorrichtung zur Entfernung eines Krankheitsherdes bekannt, die aus einer äußeren Versorgungskapillare für einen Druckflüssigkeitsstrom und einer inneren Entsorgungskapillare für einen Flüssigkeitssaugstrom besteht, wobei beide Kapillaren auf einer gemeinsamen Achse und ineinander geschobeb angeordnet sind. Am distalen Ende der Versorgungskapillare befindet sich eine Drossel.
Diese Vorrichtungen haben den Nachteil, dass der Druckflüssigkeitsstrom direkt auf das Gewebe trifft und erst dann zusammen mit den abgetrennten Gewebeteilen abgesaugt wird. Dadurch das gesunde Gewebe in unvertretbarer Weise belastet, was sich nachteilig auf den anschließenden Genesungsprozess auswirkt.

Der Erfindung liegt daher die Aufgabe zu Grunde, eine Vorrichtung der vorliegenden Art zu entwickeln, die das gesunde Gewebe schonender behandelt und die multifunktional ausgebildet ist.

Diese Aufgabe wird durch die kennzeichnenden Merkmale des Anspruchs 1 gelöst. Zweckdienliche Ausgestaltungen der Erfindung ergeben sich aus den Merkmalen der Ansprüche 2 bis 5.

Die Erfindung beseitigt die genannten Nachteile des Standes der Technik.
Darüber hinaus ist die Vorrichtung vielseitig und überall dort einsetzbar, wo Gewebe auch zum Zwecke des Austausches oder zur Probe entnommen werden muß, unabhängig davon, ob die Operation am offenen Körper oder in einer Körperkammer erfolgt. Der besondere Vorteil liegt dabei insbesondere darin, daß die Operation in einer hohen Qualität und in einer für das gesundene Gewebe äußerst schonenden Behandlung durchgeführt werden kann. Das ist in der Hauptsache auf die Vermeidung eines nach vorn gerichteten Hydrostrahls und damit von Druck und möglichen Turbulenzen in der Körperkammer zurückzuführen. Die retrograde Wirkrichtung des Hydrostrahls schont das gesunde Gewebe in besonderer Weise. Es ist auch von Vorteil, wenn in kleinsten Körperkammern z.B. in der Neurochirurgie oder in der Ophthalmologie ein Volumenausgleich für das entnommene Gewebe vorgenommen wird.
Es ist weiter von Vorteil, daß die Vorrichtung multifunktional ausgeführt ist und mehrere Funktionselemente in sich vereint. Das schont das gesunde Gewebe und vereinfacht und verkürzt die Operation.

Die Erfindung soll nachstehend an Hand eines Ausführungsbeispieles näher erläutert werden.

Dazu zeigen
- Fig. 1:: eine schematisch und vereinfacht dargestellte Hydro-Jet-Anlage und
- Fig. 2:: eine erfindungsgemäße Vorrichtung.

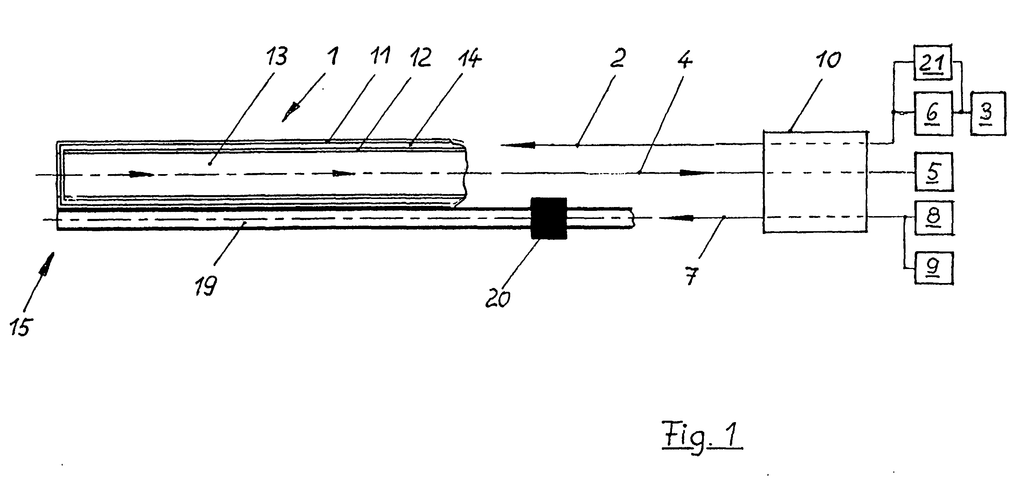

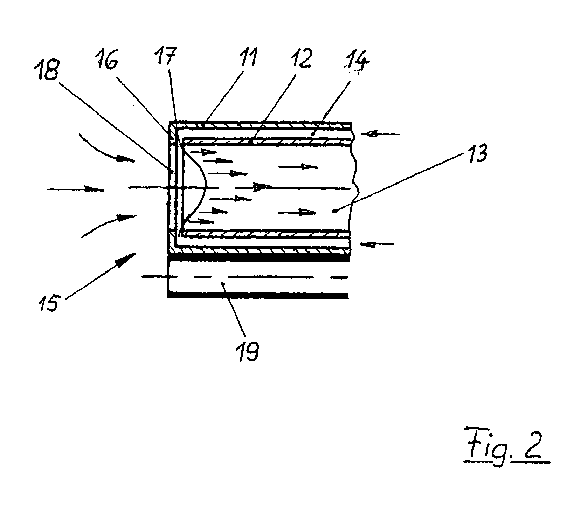

Gemäß der Fig. 1 besitzt die Hydro-Jet-Anlage in der Hauptsache eine erfindungsgemäße Vorrichtung 1 zum Entfernen von Krankheitsherden, die über eine Versorgungsleitung 2 mit einem Druckstromerzeuger 3 sowie über eine Entsorgungsleitung 4 mit einem Unterdruckstromerzeuger 5 verbunden ist. Dabei ist dem Druckstromerzeuger 3 ein zuschaltbarer Pulserzeuger 6 zugeordnet. Die Vorrichtung 1 ist weiterhin mit einer zweiten Versorgungsleitung 7 ausgerüstet, die mit einer Hydropumpe 8 und mit einer alternativ zuschaltbaren Zusatzeinheit 9, wie beispielsweise einem Hydro-Druck- oder Hydro-Jet-System oder einem Lasersystem, verbunden ist. Die Versorgungsleitungen 2, 7 und die Entsorgungsleitung 4 durchlaufen eine Steuer- und Regeleinheit 10, die vom Chirurgen manuell betätigbar ist.
Die eigentliche Vorrichtung 1 zum Entfernen von Krankeitsherden besteht, wie insbesondere aus der Fig. 2 hervorgeht, vorzugsweise aus einer äußeren Versorgungskapillare 11, die von einer inneren Entsorgungskapillare 12 durchdrungen ist. Die innere Entsorgungskapillare 12 besitzt einen Entsorgungskanal 13 und ist mit der Entsorgungsleitung 4 des Unterdruckstromerzeugers 5 verbunden. Beide Kapillaren 11 und 12 liegen auf einer gemeinsamen Achse. Durch eine entsprechende Wahl des Innendurchmessers der Versorgungskapillare 11 und des Außendurchmessers der Entsorgungskapillare 12 wird ein ringspaltförmiger Versorgungskanal 14 mit einem definierten Lichtenmaß ausgebildet, der mit der Versorgungsleitung 2 des Druckstrom- erzeugers 3 verbunden ist. Dabei bestimmt sich das definierte Lichtenmaß nach dem gewünschten Verhältnis der Querschnittsflächen des ringspaltförmigen Versorgungskanals 14 und des Entsorgungskanals 13 der Entsorgungskapillare 12. Am proximalen Ende der Vorrichtung 1 sind die Versorgungskapillare 11 und die Entsorgungskapillare 12 zu einer speziellen Hydromembran-Düse 15 ausgebildet. Dazu ist die Stirnseite der äußeren Versorgungskapillare 11 durch einen Frontring 16 abgedeckt und die innere Entsorgungskapillare 12 in der Länge um ein vorbestimmtes Maß gegenüber der äußeren Versorgungskapillare 11 zurückgesetzt. Die Längendifferenz zwischen der Versorgungskapillare 11 und der Entsorgungskapillare 12 ist größer als die Wandstärke des Frontringes 16, so daß sich zwischen der Versorgungskapillare 11 und der Entsorgungskapillare 12 ein radialer Drosselringspalt 17 ergibt. Dabei besitzt zum Zwecke einer gewünschten Drosselwirkung der radiale Drosselringspalt 17 eine geringere Querschnittsfläche als die Querschnittsfläche des ringspaltförmigen Versorgungskanales 14. Der Frontring 16 weist eine mittige Düsenöffnung 18 mit einem Durchmesser auf, der mit einem bestimmten Toleranzbereich auf den Innendurchmesser der Entsorgungskapillare 12 abgestimmt ist.
Die Vorrichtung 1 zum Entfernen eines Krankheitsherdes besitzt weiterhin eine separate Versorgungskapillare 19, in dem ein Drucksensor 20 angeordnet ist und der mit der Versorgungsleitung 7 der Hydropumpe 8 oder der Zusatzeinheit 9 verbunden ist. Diese Versorgungskapillare 19 ist vorzugsweise im starren Verbund mit der äußeren Versorgungskapillare 11 gestaltet oder als eine separate Volumenausgleichskapillare ausgebildet. Dabei besitzt diese Versorgungskapillare 19 die gleiche Länge wie die Versorgungskapillare 11.

Zum Entfernen einer eingetrübten Augenlinse im Austausch mit einer künstlichen Linse wird zunächst in herkömmlicher Weise das Auge mit einem Tunnelschnitt durchtrennt und anschließend die Vorderkapsel durch einen möglichst kreisrunden intakten Kapsulorhexis geöffnet. Durch diese Öffnung wird die Vorrichtung 1 mit ihrer Versorgungskapillare 11 für den Druckstromerzeuger 3 und ihrer Versorgungskapillare 18 für den Volumenausgleich in die erkrankte Linse geschoben. Danach gibt der Chirurg durch die Aktivierung des Druckstromerzeugers 3 einen hydraulischen Versorgungstrom in Richtung der Vorrichtung 1 frei, dessen Strom- und Druckwerte an der Steuer- und Regeleinheit 10 voreingestellt sind. Gleichzeitig aktiviert der Chirurg den Unterdruckstromerzeuger 5 und erzeugt einen Entsorgungsstrom in vorbestimmter Strom- und Druckstärke in Richtung des Unterdruckstromerzeugers 5. Damit fließt ein Hydrostrom unter Druck vom Druckstromerzeuger 3 durch die Versorgungsleitung 2 in den ringspaltförmigen Versorgungskanal 14, durchströmt den radialen Drosselringspalt 17 und tritt danach ins Freie. Hier wird er vom Entsorgungsstrom des Unterdruckstromerzeugers 5 erfaßt und durch den Entsorgungskanal 13 der Entsorgungskapillare 12 und die Entsorgungsleitung 4 in retrograder Richtung zum Unterdruckstromerzeuger 5 befördert. Durch die richtige Wahl der an der Steuer- und Regeleinheit 10 eingestellten Strom- und Druckwerte beider Ströme entstehen unterschiedliche Strömungsgeschwindigkeiten zwischen dem Versorgungsstrom und dem Entsorgungsstrom und damit an der Hydromembran-Düse 15 ein Unterdruckbereich, der an der Mündung der inneren Entsorgungskapillare 12 eine kegelförmige Hydromembrane und damit Anzugskräfte auf das unmittelbare Umfeld der Hydromembran-Düse 15 erzeugt. Die Größe dieser Anzugskräfte ist an der Steuer- und Regeleinheit 10 veränder- und einstellbar.
Diese Anzugskräfte lösen das erkrankte Gewebe der Augenlinse von dem gesunden Gewebe der benachbarten Augenbestandteile und zerkleinern gleichzeitig die abgelösten Linsenteile und transportieren sie zur Mündung der Hydromembran-Düse 15. Kleinere Linsenreste treten ungehindert in den Entsorgungskanal 13 ein, größere Linsenreste treffen auf den Frontring 16 der äußeren Versorgungskapillare 11 auf und werden dort weiter auf eine transporttaugliche Größe zerkleinert. Die abtragende Wirkung der Anzugskräfte kann dadurch verstärkt werden, daß der Versorgungsstrom durch die Aktivierung des Pulserzeugers 6 pulsierend ausgeführt wird.
Zum Ausgleich des Volumenverlustes innerhalb der Linsenkammer wird die Hydropumpe 8 betätigt, die der Augenkammer in dem Maße Flüssigkeit zuführt, wie erkranktes Linsengewebe abgeführt wird. Dieser Volumenausgleich kann kontinuierlich und über den Drucksensor 20 oder manuell gesteuert ablaufen. Im alternativen Betrieb zum Volumenausgleich kann beispielsweise eine Zusatzeinheit 9 in Form einer Water-Jet-Anlage eingesetzt werden, die über die Versorgungskapillare 19 einen Wasser- oder Laser strahl für weitere, die Operation begleitende Maßnahmen erzeugt.
Zweckmäßiger Weise ist in die Versorgungsleitung 2 ein Heizgerät 21 eingebunden, um den Hydrostrom einer gewünschten Temperatur anzugleichen.

### Aufstellung der Bezugszeichen

- 1: Vorrichtung
- 2: Versorgungsleitung
- 3: Druckstromerzeuger
- 4: Entsorgungsleitung
- 5: Unterdruckstromerzeuger
- 6: Pulserzeuger
- 7: Versorgungsleitung
- 8: Hydropumpe
- 9: Zusatzeinheit
- 10: Steuer- und Regeleinheit
- 11: Versorgungskapillare
- 12: Entsorgungskapillare
- 13: Entsorgungskanal
- 14: ringspaltförmiger Versorgungskanal
- 15: Hydromembran-Düse
- 16: Frontring
- 17: radialer Drosselringspalt
- 18: Düsenöffnung
- 19: Versorgungskapillare
- 20: Drucksensor
- 21: Heizgerät

## Patentansprüche

1. Vorrichtung zum Entfernen von Krankheitsherden in der Human- und Veterinärmedizin, bestehend aus einer äußeren Versorgungskapillare (11) mit einem ringförmigen Versorgungskanal (14) für einen Druckstrom und einer inneren, in die äußere Versorgungskapillare (11) eingeschobenen Entsorgungskapillare (12) mit einem Entsorgungskanal (13) für einen Saugstrom, wobei der Versorgungskanal (14) der Versorgungskapillare (11) an seinem distalen Ende eine Drossel besitzt und mit dem Entsorgungskanal (13) der Entsorgungskapillare (12) funktionell verbunden ist,
**dadurch gekennzeichnet, dass** der Entsorgungskanal (13) an seinem distalen Ende offen ausgeführt ist und die Drossel am distalen Ende der Versorgungskapillare (11) als ein radialer Drosselringspalt (17) ausgebildet ist und der Versorgungskanal (14)über diesen Drosselringspalt (17)in den Entsorgungskanal (13) der Entsorgungskapillare (12) mündet, so dass bei unterschiedlichen Strömungsgeschwindigkeiten zwischen dem Versorgungsstrom und dem Entsorgungsstrom an der Mündung der inneren Entsorgungskapillare (12) eine kegelförmige und in Saugrichtung zeigende Hydromembran, erzeugt werden kann.

2. Vorrichtung nach Anspruch 1,
**dadurch gekennzeichnet, daß** der radiale Drosselringspalt (17) durch das in der Länge zurückgesetzte Ende der inneren Entsorgungskapillare (12) und durch einen auf die äußere Versorgungskapillare (11) aufgesetzten Frontring (16) gebildet wird.

3. Vorrichtung nach Anspruch 2,
**dadurch gekennzeichnet, daß** der Frontring (16) eine Düsenöffnung (18) besitzt, dessen Durchmesser dem Durchmesser des Entsorgungskanals (13) entspricht.

4. Vorrichtung nach Anspruch 1,
**dadurch gekennzeichnet, daß** die äußere Versorgungskapillare (11) mit einer weiteren und parallel verlaufenden Versorgungskapillare (19) für einen erforderlichen Volumenausgleich in der Körperkammer oder für einen alternativen Operationsschritt verbunden ist.

5. Vorrichtung nach Anspruch 4,
**dadurch gekennzeichnet, daß** in der zweiten Versorgungskapillare (19) ein Drucksensor (20) angeordnet ist.

## Claims

1. Device for removing disease foci in human and veterinary medicine, consisting of an outer supply capillary (11) with an annular supply passage (14) for a pressure flow and an inner discharge capillary (12) with a discharge passage (13) for a suction flow inserted in the outer supply capillary (11), which supply passage (14) of the supply capillary (11) has a throttle at its distal end and is actively connected to the discharge passage (13) of the discharge capillary (12),
**characterised in that** the discharge passage (13) is open at its distal end and the throttle is provided in the form of a radial throttle annular gap (17) at the distal end of the supply capillary (11), and the supply passage (14) opens via this throttle annular gap (17) into the discharge passage (13) of the discharge capillary (12) so that a cone-shaped hydro-membrane pointing in the suction direction can be created when there is a difference in flow speeds between the supply flow and the discharge flow at the opening of the inner supply capillary (12).

2. Device as claimed in claim 1,
**characterised in that** the radial throttle annular gap (17) is formed by the end of the inner discharge capillary (12) which is recessed lengthways and by a front ring (16) placed on the outer supply capillary (11).

3. Device as claimed in claim 2,
**characterised in that** the front ring (16) has a nozzle orifice (18), the diameter of which matches the diameter of the discharge passage (13).

4. Device as claimed in claim 1,
**characterised in that** the outer supply capillary (11.) is connected to another supply capillary (19) extending parallel as a means of achieving the required compensation of volume in the body chamber or for the purpose of an alternative operating step.

5. Device as claimed in claim 4,
**characterised in that** a pressure sensor (20) is disposed in the second supply capillary (19).

## Revendications

1. Dispositif pour éliminer les foyers de maladie en médecine humaine et vétérinaire, constitué d'un capillaire d'alimentation externe (11) avec un canal d'alimentation annulaire (14) pour un jet sous pression et un capillaire d'élimination (12) interne inséré dans le capillaire d'alimentation externe (11), avec un canal d'élimination (13) pour un débit d'aspiration, le canal d'alimentation (14) du capillaire d'alimentation (11) possédant à son extrémité distale un étranglement en étant relié de façon fonctionnelle avec le canal d'élimination (13) du capillaire d'élimination (12),
**caractérisé en ce que**
le canal d'élimination (11) est ouvert à son extrémité distale et l'étranglement à l'extrémité distale du capillaire d'alimentation (11) est formé en fente annulaire d'étranglement radial (17), et **en ce que** le canal d'alimentation (14) débouche par cette fente annulaire d'étranglement (17) dans le canal d'élimination (13) du capillaire d'élimination (12), de manière qu'à des vitesses d'écoulement différentes entre le débit d'alimentation et le débit d'élimination au débouché du capillaire d'élimination interne (12), on puisse produire une hydromembrane en forme de cône et montrant la direction d'aspiration.

2. Dispositif selon la revendication 1,
**caractérisé en ce que**
la fente annulaire d'étranglement radial (17), est formée à travers l'extrémité longitudinalement en retrait du capillaire d'élimination interne (12) et à travers un anneau frontal (16) disposé sur le capillaire d'alimentation externe (11).

3. Dispositif selon la revendication 2,
**caractérisé en ce que**
l'anneau frontal (16) possède une ouverture de buse (18) dont le diamètre correspond au diamètre du canal d'élimination (13).

4. Dispositif selon la revendication 1,
**caractérisé en ce que**
le capillaire d'alimentation externe (11) est relié à un autre capillaire d'alimentation (19), de direction parallèle, en vue d'une compensation volumique nécessaire dans la chambre corporelle, ou en vue d'une autre étape opératoire.

5. Dispositif selon la revendication 4,
**caractérisé en ce qu'**
un capteur de pression (20) est disposé dans le second capillaire d'alimentation (19).
